# EUROPÄISCHE PATENTANMELDUNG

(11) **EP 4 596 667 A1**
(43) Veröffentlichungstag der Anmeldung: **06.08.2025**
(21) Anmeldenummer: 24154832.0
(22) Anmeldetag: 30.01.2024
(51) Int. Cl.: C12M 1/24, C12M 3/00, C12M 3/04, C12M 1/00, C12M 1/26, C12M 1/12, C12M 1/34

(54) **BIOREAKTOR UND PROZESSLEITSYSTEM**

(71) Anmelder: Green Elephant Biotech GmbH, 35394 Gießen (DE)
(72) Erfinder: Eichmann, Joel, 89160 Dornstadt (DE); Käßer, Lukas, 35460 Staufenberg (DE); Boshof, Bjön, 35394 Gießen (DE); Kaiser, Nils, 35390 Gießen (DE)
(74) Vertreter: Metten, Karl-Heinz

(57) **Zusammenfassung**

Die vorliegende Erfindung betrifft einen Bioreaktor zum Kultivieren von Zellen, umfassend ein Gehäuse, ein Reaktorgefäß, mindestens eine Leitung für den Transfer von Gasen oder Gasgemischen zu dem und/oder aus dem Reaktorgefäß mit mindestens einer reversibel oder irreversibel verschließbaren Verschlusseinheit, mindestens eine Leitung für den Transfer flüssiger Systeme zu dem und/oder aus dem Reaktorgefäß mit mindestens einer reversibel oder irreversibel verschließbaren Verschlusseinheit und mindestens ein Rotationsmodul, ausgelegt und eingerichtet für die Bewegung des Reaktorgefäßes um dessen Längsachse. Ferner betrifft die Erfindung ein Prozessleitsystem umfassend mindestens einen erfindungsgemäßen Bioreaktor.

## Beschreibung

Die vorliegende Erfindung betrifft einen Biorektor sowie ein Prozessleitsystem enthaltend den Bioreaktor.

Bioreaktoren als solche sind dem Fachmann bekannt.

Aus der DE 693 32 374 T2 geht ein Bioreaktor zum Kultivieren von Humanzellen hervor, umfassend eine Zellkulturkammer mit einer Vielzahl von Einlassöffnungen und einer Vielzahl von im Wesentlichen äquidistanten Auslassöffnungen, Mittel zum Perfundieren eines flüssigen Zellkulturmediums durch die Zellkulturkammer, eine gaspermeable Membran zum In-Kontakt-Bringen des flüssigen Zellkulturmediums mit einer Sauerstoffquelle und Mittel zum Ernten nicht adhärenter Zellen aus der Zellkulturkammer durch die Strömung des Kulturmediums. Dieser Bioreaktor soll das Unterhalten und die Proliferation von humanen Stammzellen und/oder frühen humanen hämatopoetischen Zellen, einschließlich komplexer Kulturen von Primärzellen, in einer Weise erlauben, dass das Ernten der in dem Bioreaktor produzierten Zellen ohne Unterbrechung der Kultur möglich ist.

Die DE 10 2017 208 758 A1 betrifft eine Vorrichtung zur Kultivierung von Zellen, umfassend eine Mischvorrichtung und einen von einer Reaktorwand begrenzten Reaktionsraum, die mindestens einen Anschluss für die Zugabe von Gas und/ oder Flüssigkeit aufweist. Der Reaktionsraum hat so ausgestaltet zu sein, dass entweder dessen inneres Volumen, bezogen auf ein minimales inneres Anfangsvolumen, um mindestens 500% vergrößerbar ist oder dass eine horizontale Querschnittsfläche des Reaktionsraums von unten nach oben zunimmt. Mit einer derartigen Vorrichtung soll das Überführen der Zellen in mehrere Gefäße verschiedener Größe bei der Zellexpansion vermieden werden können.

Die DE 10 2018 123 553 A1 offenbart einen Bioreaktor für die Kultivierung biologischer Zellen, umfassend einen Behälter zur Aufnahme eines Kultivierungsmediums mit einer Vielzahl an darin angeordneten Substratfilamenten, die für eine temporäre adhärente Kopplung der biologischen Zellen eingerichtet sind. Hierfür haben die Substratfilamente mit einer Oberflächenschicht versehen zu sein, die zwischen einem Adhärenzzustand, in dem die biologischen Zellen adhärent an die Oberflächenschicht koppelbar sind, und einem Freigabezustand, in dem die adhärente Kopplung der biologischen Zellen mit der Oberflächenschicht im Vergleich zum Adhärenzzustand verringert ist, schaltbar ist. Mit dem Bioreaktor der DE 10 2018 123 553 A1 soll eine Kultivierung biologischer Zellen mit einer erhöhten Effizienz und Ausbeute und/oder mit einer schonenden Ablösung von Zellen von dem Kultivierungssubstrat gelingen.

Die DE 10 2019 100 434 A1 befasst sich mit einem Bioreaktor zur Kultivierung von Mikroorganismen und Zellen tierischer oder pflanzlicher Herkunft, mit einer auf der Innenfläche einer flexiblen Wand des Bioreaktors angeordneten Filtertasche, die außenseitig durch die Wand des Bioreaktors und innenseitig durch eine Filtertaschenwand begrenzt und bei der zwischen der Wand des Bioreaktors und dem Filtermedium ein Abstandshalter angeordnet ist. Hierbei hat die innenseitige Filtertaschenwand zumindest teilweise durch ein Filtermedium gebildet zu sein und der Abstandshalter hat wenigstens eine Durchgangsöffnung aufzuweisen, durch die ein Verbindungsabschnitt des Filtermediums hindurchzuragen und direkt mit der Wand des Bioreaktors verbunden zu sein hat. Auf diese Weise soll ein zur Perfusion geeigneter Bioreaktor verfügbar werden, der auch für große Volumina in der Größenordnung von 200 Litern Totalvolumen oder mehr einsetzbar ist.

Aus der EP 3 330 368 B1 geht ein Verfahren zur Kultivierung von Zellen in einer Zellkulturkammer eines Bioreaktorsystems hervor, umfassend i) das Einbringen einer Zellprobe in eine erste Zellkulturkammer eines ersten Zellmoduls des Bioreaktorsystems, wobei das erste Zellmodul der ersten Zellkulturkammer einen Einlass, einen Auslass und eine Anschlussöffnung aufweist und ferner mindestens ein männliches Klemmverbindungsstück mit einer Vertiefung und mindestens ein weibliches Verbindungsstück mit einem erhabenen Abschnitt umfasst, wobei das männliche und das weibliche Verbindungsstück auf derselben Fläche des Zellmoduls angeordnet sind; ii) das Einbringen eines Zellkulturmediums, das die Migration fördert, in eine zweite Zellkulturkammer eines zweiten Zellmoduls, wobei das zweite Zellmodul der zweiten Zellkulturkammer einen Einlass, einen Auslass und eine Anschlussöffnung aufweist, und wobei eine Membran zwischen dem offenen Anschluss des ersten Zellmoduls und dem offenen Anschluss des zweiten Zellmoduls positioniert ist und wobei das erste Zellmodul und das zweite Zellmodul abdichtend ineinandergreifen, wodurch die Membran zwischen dem ersten und dem zweiten Modul befestigt wird und wobei die Vertiefung in der mindestens einen männlichen Klemmverschraubung des ersten Moduls und der erhabene Abschnitt in der mindestens einen weiblichen Verschraubung des zweiten Zellmoduls unter Verwendung einer Druck- und Drehbewegung abdichtend in Eingriff gebracht werden. Mit diesem Verfahren soll die Co-Kultivierung mehrerer Zelltypen in einer dynamischen Umgebung, in der die verschiedenen Zelltypen biochemisch kommunizieren können und dennoch physisch trennbar sind, gelingen.

Die WO 2013/ 124326 A1 betrifft eine Bioreaktoranlage mit einem mit einem Feststoffabscheider verbundenen Bioreaktor. Der Feststoffabscheider umfasst einen durchströmten sterilisierbaren Kunststoffbeutel und innerhalb des Kunststoffbeutels im oberen Bereich ein oder mehrere Einbauten zum Abziehen eines von den Feststoffen getrennten Erntestroms aus einem Erntestromsammelbereich. Im oberen Segment eines mittleren Bereiches hat ein Abscheidebereich mit einer Abscheidefläche vorzuliegen, die während des Betriebs mit einem Winkel von 0° bis 80° zur Horizontalen geneigt ist. Ferner haben im unteren Segment des mittleren Bereiches eine oder mehrere Durchführungen oder Einbauten zur Strömungsverteilung des Reaktorgemisches vorzuliegen. Schließlich hat im unteren Bereich ein sich nach unten verjüngender Feststoffsammelbereich zum Sammeln der Feststoffe mit Hilfe der Schwerkraft vorzuliegen. Mit der Anlage der WO 2013/ 124326 A1 soll eine effiziente Methode zur Rückhaltung und Rückführung von tierischen und pflanzlichen Zellen in einem kontinuierlich oder absatzweise betriebenen Verfahren bereitgestellt werden, die der Empfindlichkeit der Zellen hinsichtlich mechanischer Scherbeanspruchung und ausreichender Versorgung der Zellen mit Nährstoffen Rechnung trägt.

In der CA 2 566 841 C wird abgestellt auf einen Bioreaktor, umfassend eine Membranträgerstruktur und eine hochporöse Membran, die auf der Membranträgerstruktur aufliegt. Die Membran weist eine Nährstoffseite und eine sogenannte Gasseite auf. Ferner hat die Membran eine immobilisierte Bioschicht zu enthalten. Die Membran hat derart eingerichtet und ausgelegt zu sein, dass sie die Diffusion einer Nährlösung von der Nährstoffseite zu der immobilisierten Bioschicht ohne äußeren Druck ermöglicht. Schließlich haben die Membrane in Paaren auf der Membranträgerstruktur angeordnet zu sein und einen Innenbereich zu definieren. Der Bioreaktor der CA2 566 841 C soll kostengünstig und langlebig sein und höhere Biokonversionsraten als herkömmliche Systeme ermöglichen.

Aus der CA 3 086 283 A1 ist ein Bioreaktor zum Kultivieren von Zellen bekannt, umfassend ein Basisteil mit einer ersten Kammer enthaltend einen Rührer und eine erste zentrale Säule, die abnehmbar an dem Basisteil angebracht ist, wobei die erste zentrale Säule mindestens einen Teil einer äußeren zweiten Kammer zum Züchten von Zellen und eine innere dritte Kammer zum Zurückführen des Fluidstroms von der zweiten äußeren Kammer zu der ersten Kammer bildet. Auf diese Weise soll ein modular aufgebauter Bioreaktor bereitgestellt werden, mit dem ein oder mehrere strukturierte Festbetten verwendet werden können, um den Herstellungsprozess zu vereinfachen und gleichzeitig hervorragende Zellkultivierungsergebnisse in Sachen Homogenität und Wiederholbarkeit zu erzielen.

Aus der DE 42 00 446 A1 geht schließlich ein Bioreaktor, der mit einer Zellträgeranordnung ausgestattet ist, hervor, mit der in optimaler Weise Biomaterial wie Zellen produziert werden können. Hierfür hat die Zellträgeranordnung wenigstens doppelwandig ausgebildet zu sein und aus wenigstens zwei Zuschnitten aus einem Flachmaterial zu bestehen, wobei die Zuschnitte zwischen sich wenigstens einen Spalt bzw. einen spaltartigen Bereich zu bilden haben.

Der vorliegenden Erfindung hat die Aufgabe zugrunde gelegen, einen Bioreaktor verfügbar zu machen, der nicht mit den Nachteilen des Stands der Technik behaftet ist und der insbesondere problemlos und sicher gehandhabt werden kann und mit dem auch adhärente Zellen kultiviert werden können.

Demgemäß wurde ein Bioreaktor, insbesondere Single-use Bioreaktor, zum Kultivieren von, insbesondere adhärenten, Zellen gefunden, umfassend
- ein Gehäuse mit einer Gehäusewandung und einem Gehäuseboden sowie,
- ein Reaktorgefäß mit einem Reaktorboden und einem gegenüberliegenden Reaktorende sowie einer sich von dem Reaktorboden zum gegenüberliegenden Reaktorende erstreckenden Längsachse,
- mindestens eine Leitung für den Transfer von Gasen oder Gasgemischen zu dem und/oder aus dem Reaktorgefäß mit mindestens einer reversibel oder irreversibel verschließbaren Verschlusseinheit,
- mindestens eine Leitung für den Transfer flüssiger Systeme zu dem und/oder aus dem Reaktorgefäß mit mindestens einer reversibel oder irreversibel verschließbaren Verschlusseinheit und
- mindestens ein Rotationsmodul, ausgelegt und eingerichtet für die Bewegung des Reaktorgefäßes um die Längsachse.

Ganz besonders bevorzugt sind dabei solche erfindungsgemäßen Bioreaktoren, insbesondere Single-use Bioreaktor, zum Kultivieren von, insbesondere adhärenten, Zellen, umfassend
- ein Gehäuse mit einer Gehäusewandung und einem Gehäuseboden sowie
- ein Reaktorgefäß mit einem Reaktorboden und einem gegenüberliegenden Reaktorende sowie einer sich von dem Reaktorboden zum gegenüberliegenden Reaktorende erstreckenden Längsachse, wobei die Längsachse gegenüber der Horizontalen einen Neigungswinkel aufweist,
- mindestens eine Leitung für den Transfer von Gasen oder Gasgemischen zu dem und/oder aus dem Reaktorgefäß mit mindestens einer reversibel oder irreversibel verschließbaren Verschlusseinheit,
- mindestens eine Leitung für den Transfer flüssiger Systeme zu dem und/oder aus dem Reaktorgefäß mit mindestens einer reversibel oder irreversibel verschließbaren Verschlusseinheit und
- mindestens ein Rotationsmodul, ausgelegt und eingerichtet für die Bewegung des Reaktorgefäßes um die Längsachse.

In dem Gehäuse liegen insbesondere das Reaktorgefäß, die mindestens eine Leitung für den Transfer von Gasen oder Gasgemischen zu dem und/oder aus dem Reaktorgefäß, die mindestens eine Leitung für den Transfer flüssiger Systeme zu dem und/oder aus dem Reaktorgefäß und das mindestens eine Rotationsmodul oder ein Bestandteil hiervon vor. Die Verschlusseinheit der Leitung für den Transfer von Gasen oder Gasgemischen liegt bevorzugt in oder an der Gehäusewandung oder besonders bevorzugt außerhalb des Gehäuses vor. Die Verschlusseinheit der Leitung für den Transfer flüssiger Systeme zu dem und/oder aus dem Reaktorgefäß kann ebenfalls außerhalb des Gehäuses vorliegen, ist jedoch bevorzugt in oder an der Gehäusewandung angebracht.

In zweckmäßigen Ausführungsformen des erfindungsgemäßen Bioreaktors liegt neben der mindestens einen Leitung für den Transfer von Gasen oder Gasgemischen mit der mindestens einen Verschlusseinheit zu dem Reaktorgefäß auch mindestens eine Leitung für den Transfer von Gasen oder Gasgemischen mit der mindestens einen Verschlusseinheit aus dem Reaktorgefäß vor. Die mindestens eine Leitung für den Transfer von Gasen oder Gasgemischen zu dem Reaktorgefäß stellt vorzugsweise eine Luft-, Sauerstoff-, Stickstoff- und/oder eine Kohlendioxidzuleitung dar. Beispielsweise kann in einer Ausführungsform eine Sauerstoffleitung zu dem Reaktorgefäß neben einer weiteren Leitung für den Transfer von z.B. Stickstoff vorgesehen sein. Selbstverständlich können auch beliebige Gasgemische über eine Leitung zu dem Reaktorgefäß transferiert werden.

In einer weiteren, besonders zweckmäßigen Ausführungsform des erfindungsgemäßen Bioreaktors liegt neben der mindestens einen Leitung für den Transfer flüssiger Systeme mit der mindestens einen Verschlusseinheit zu dem Reaktorgefäß auch mindestens eine Leitung für den Transfer flüssiger Systeme mit der mindestens einen Verschlusseinheit aus dem Reaktorgefäß vor. Hierbei stellt diese mindestens eine Leitung für den Transfer flüssiger Systeme aus dem Reaktorgefäß, bevorzugt wenn als Flasche und besonders bevorzugt als Zellkultur-Flasche ausgebildet, eine Abführleitung für kultivierte Zellen dar. In den Leitungen für den Transfer von Gasen oder Gasgemischen hat sich in vielen Fällen als sehr vorteilhaft erwiesen, mindestens ein Sterilfilter vorzusehen bzw. zu integrieren. In einer weiteren Ausführungsform können auch die Leitungen für den Transfer flüssiger Systeme mit einem Sterilfilter ausgestattet sein.

Das Gehäuse des erfindungsgemäßen Bioreaktors ist vorzugsweise nicht öffenbar und/oder, insbesondere und, gas- und flüssigkeitsdicht ausgestaltet. Besonders bevorzugt ist das Gehäuse nicht öffenbar sowie gas- und flüssigkeitsdicht ausgestaltet.

Auf die vorangehend geschilderte Weise kann jegliche Kontamination der Umgebung mit einem Höchstmaß an Sicherheit ausgeschlossen werden. Auch gestattet dies in besonders zuverlässiger Weise die Handhabung des erfindungsgemäßen Bioreaktors als Modul bzw. Einwegkassette, die sich unproblematisch und sicher in bestehende Prozessleitsysteme oder Prozessanlagen einbinden lässt. Zum Abbau von Überdruck in diesen Bioreaktoren kann in einer bevorzugten Ausführungsform das Gehäuse, insbesondere die Gehäusewandung, mit mindestens einem Sterilfilter ausgestattet sein.

Während mit dem Rotationsmodul zuvorderst das Reaktorgefäß um seine Längsachse während des Kultivierungsprozesses gedreht werden soll, ist in einer bevorzugten Ausführungsform ein mindestens in Teilen innerhalb des Gehäuses vorliegendes Agitationsmodul, welches das Reaktorgefäß erschüttern soll, mit einer insbesondere außerhalb des Gehäuses vorliegenden Antriebseinheit, insbesondere reversibel, verbunden oder verbindbar. In einer alternativen Ausführungsform liegt das Agitationsmodul außerhalb des Gehäuses des Bioreaktors vor. In diesem Fall wird das gesamte Gehäuse, einschließlich Reaktorgefäß einer Agitation ausgesetzt. Mit Hilfe des Agitationsmoduls soll insbesondere der Prozess des Erntens der kultivierten Zellen initiiert bzw. erleichtert werden.

In einer besonders geeigneten Ausführungsform stellt das Agitationsmodul ein Vibrationsmodul dar oder umfasst dieses. Das Agitations- sowie insbesondere das Vibrationsmodul kann dabei in einer zweckmäßigen Ausführungsform eine Exzenterscheibe darstellen oder eine solche umfassen.

Der erfindungsgemäße Bioreaktor umfasst in einer besonders zweckmäßigen Ausführungsform mindestens eine in dem Gehäuse oder vorzugsweise außerhalb des Gehäuses vorliegende erste Antriebseinheit, ausgelegt und eingerichtet zum Antrieb des Rotationsmoduls. Alternativ sowie insbesondere zusätzlich verfügt dieser erfindungsgemäße Bioreaktor über mindestens eine in dem Gehäuse oder vorzugsweise außerhalb des Gehäuses vorliegende zweite Antriebseinheit, ausgelegt und eingerichtet zum Antrieb des Agitationsmoduls.

In einer weiteren bevorzugten Ausgestaltung des erfindungsgemäßen Bioreaktors ist vorgesehen, dass die mindestens eine Leitung für den Transfer von Gasen oder Gasgemischen zu dem und/oder aus dem Reaktorgefäß mit mindestens einer reversibel oder irreversibel verschließbaren Verschlusseinheit in einen in der, an der oder benachbart zur Gehäusewand vorliegenden ersten Sterilkonnektor mündet. Dabei kann alternativ sowie insbesondere zusätzlich vorgesehen sein, dass die mindestens eine Leitung für den Transfer von Gasen oder Gasgemischen zu dem und/oder aus dem Reaktorgefäß mit mindestens einer reversibel verschließbaren Verschlusseinheit, insbesondere benachbart zur Gehäusewand, mit einem Sterilfilter ausgestattet ist. Ferner hat es sich für bevorzugte Ausführungsformen des erfindungsgemäßen Bioreaktors als vorteilhaft erwiesen, dass die mindestens eine Leitung für den Transfer flüssiger Systeme zu dem und/oder aus dem Reaktorgefäß mit mindestens einer reversibel oder irreversibel verschließbaren Verschlusseinheit in einen in der, an der oder benachbart zur Gehäusewand vorliegenden zweiten Sterilkonnektor mündet.

Der erfindungsgemäße Bioreaktor umfasst in einer besonders bevorzugten Ausführungsform ferner mindestens eine interne oder externe, insbesondere strom-, flüssigkeits- oder gasbasierte, Temperiereinheit. Für viele Anwendungen hat es sich als sehr zweckmäßig erwiesen, die mindestens eine Temperiereinheit in der mindestens einen Rotationseinheit, insbesondere in mindesten einer Rolle, zu integrieren. Dies führt zu einem sehr kompakt gebauten erfindungsgemäßen Bioreaktor. Auch kann in einer alternativen Ausführungsform vorgesehen sein, das Reaktorgefäß doppelwandig auszuführen und eine Temperierung durch Einleitung von erwärmten Flüssigkeiten durch den so gebildeten Hohlraum herbeizuführen.

Darüber hinaus kann in besonders zweckmäßigen Ausführungsformen des erfindungsgemä-βen Bioreaktors vorgesehen sein, dass dieser ferner, insbesondere in dem Gehäuse vorliegend, mindestens einen Sauerstoffsensor und/oder mindestens einen Kohlendioxidsensor und/oder mindestens einen Laktatsensor und/oder mindestens einen Glukose-sensor und/oder mindestens einen Fruktosesensor und/oder mindestens einen Temperatursensor und/oder mindestens einen pH-Sensor und/oder mindestens einen Impedanzsensor und/oder mindestens einen Permittivitätssensor und/oder mindestens einen Kapazitätssensor und/oder mindestens einen Kontaktsensor, eingerichtet und ausgelegt zur Feststellung des Öffnungsstatus der mindestens einen Verschlusseinheit, umfasst.

In einer zweckmäßigen Ausführungsform des erfindungsgemäßen Bioreaktors kann vorgesehen sein, dass ein oder mehrere Sensoren innerhalb einer Messzelle, insbesondere Durchflussmesszelle, vorliegen oder in Kontakt stehen oder bringbar sind mit einer Messzelle. Diese Messzelle liegt vorzugsweise innerhalb des Gehäuses vor. Mit der Messzelle ist es möglich, Fluid dem Reaktorgefäß zu entnehmen, um es nach Durchführung der Messung dem Reaktorgefäß wieder zuzuführen.

In dem erfindungsgemäßen Bioreaktor kommen bevorzugt mindestens ein Temperatur-, Sauerstoff-, pH-Wert-, Glukose- und/oder, insbesondere und, Laktatsensor zum Einsatz. Solche Messzellen für den erfindungsgemäßen Bioreaktor sind besonders bevorzugt, die mit einem Sauerstoff-, pH-Wert-, Glukose- und/oder, insbesondere und, Laktatsensor ausgestattet sind. Diese liegen bevorzugt in dem Gehäuse vor.

Der erfindungsgemäße Bioreaktor kann ferner ausgestattet sein mit mindestens einer ersten Pumpeinheit oder einem ersten Pumpenelement in Wirkverbindung mit der mindestens einen Leitung für den Transfer von Gasen oder Gasgemischen zu dem und/oder aus dem Reaktorgefäß sowie alternativ und insbesondere zusätzlich mit mindestens einer zweiten Pumpeinheit oder einem zweiten Pumpenelement in Wirkverbindung mit der mindestens einen Leitung für den Transfer flüssiger Systeme zu dem und/oder aus dem Reaktorgefäß. Die zweite Pumpeinheit ist dabei bevorzugt ausgelegt und eingerichtet für die Entnahme und/oder Zugabe von Prozessmedien und/oder Reagenzien und/oder kultivierten Zellen. Das erste Pumpenelement in Wirkverbindung mit der mindestens einen Leitung für den Transfer von Gasen oder Gasgemischen zu dem und/oder aus dem Reaktorgefäß liegt dabei bevorzugt in oder an der Gehäusewandung vor. Ferner kann auch das zweite Pumpenelement in Wirkverbindung mit der mindestens einen Leitung für den Transfer flüssiger Systeme zu dem und/oder aus dem Reaktorgefäß in oder an der Gehäusewandung vorliegen. Mit den vorangehend genannten Ausführungsformen lässt sich ein besonders handhabungsfreundlicher sowie zuverlässig flüssigkeits- und gasdichter erfindungsgemäßer Bioreaktor erhalten. Bei einer Pumpeinheit kann es sich z.B. um eine sogenannte Schlauch- bzw. Schlauchquetschpumpe oder um eine Membranpumpe handeln. Das Pumpenelement einer Schlauchpumpe kann dabei einen Pumpenkopf enthaltend an einem Rotor vorliegende Gleitschuhe oder Rollen umfassen. Derartige Pumpenelemente lassen sich in eine Gehäusewandung integrieren bzw. können innerhalb des Gehäuses vorliegen, wobei der Pumpenantrieb außerhalb des Gehäuses angeordnet ist und separiert werden kann. Die vorangehend geschilderte Ausgestaltung eines Pumpenelements ist insbesondere auch für den Transfer von Flüssigkeiten geeignet.

Der erfindungsgemäße Bioreaktor verfügt bevorzugt über mindestens eine zweite Pumpeinheit oder ein zweites Pumpenelement in Wirkverbindung mit der mindestens einen Leitung für den Transfer flüssiger Systeme zu dem und/oder aus dem Reaktorgefäß. Bei dieser Ausführungsform hat nicht gleichzeitig auch die mindestens eine erste Pumpeinheit oder das erste Pumpenelement für den Transfer von Gasen oder Gasgemischen zu dem und/oder aus dem Reaktorgefäß als Bestandteil des Bioreaktors vorzuliegen. Mit einer zweiten Pumpeinheit oder mit Hilfe eines zweiten Pumpenelements kann Flüssigkeit sowohl in das Reaktorgefäß hineinbefördert und auch herausbefördert werden Auch kann man für die Befüllung und die Entnahme auf zwei getrennte zweite Pumpeinheiten zurückgreifen. Die zweite Pumpeinheit ist demgemäß bevorzugt ausgelegt und eingerichtet für die Entnahme und/oder Zugabe von Prozessmedien und/oder Reagenzien und/oder kultivierten Zellen.

Ein besonders ausgeprägtes Maß an Handhabungssicherheit und Handhabungsfreundlichkeit kann auch dadurch sichergestellt werden, dass der erfindungsgemäße Bioreaktor ferner eine innerhalb oder außerhalb, insbesondere jedenfalls in Teilen innerhalb, des Gehäuses vorliegende Höhenverstelleinheit zur Einstellung des Neigungswinkels der Längsachse des Reaktorgefäßes aufweist. Die Höhenverstelleinheit stellt in sehr zweckmäßigen Ausführungsformen eine Verstellschraube oder einen Verstellmotor dar oder umfasst diese. Über die Einstellung des Neigungswinkels kann zum Beispiel Einfluss genommen werden auf den Befüllungsgrad des Reaktorgefäßes des erfindungsgemäßen Bioreaktors.

Das Reaktorgefäß des erfindungsgemäßen Bioreaktors stellt bevorzugt eine Flasche, insbesondere eine Zellkultur-Flasche, mit einem Flaschenboden und einem gegenüberliegenden Flaschenende dar bzw. umfasst diese. Die Zellkultur-Flasche umfasst in zweckmäßigen Ausführungsformen einen Bodenbereich, einen Deckelbereich und einen dazwischen angeordneten Zylinderbereich. Bevorzugt ist dabei im Zylinderbereich innenliegend eine archimedische Schraube mit einem entlang ihrer Mittelachse angeordneten flüssigkeitsdurchströmbaren Rohr, auch Mittelrohr genannt, vorgesehen. Bevorzugt liegt wenigstens eine konzentrisch um das Rohr angeordnete Wand vor, welche sich über den Zylinderbereich erstreckt.

Besonders bevorzugt sind die Zellkultur-Flaschen einstückig ausgebildet. Ferner ist besonders bevorzugt das Mittelrohr in einer Weise ausgebildet, dass es eine erste Öffnung an der Außenseite der Zellkultur-Flasche im Deckelbereich und eine zweite Öffnung im Inneren der Zellkultur-Flasche an der Unterseite des Zylinderbereiches aufweist. Darüber hinaus sind auch solche Zellkultur-Flaschen bevorzugt, bei denen die archimedische Schraube einstückig mit dem Zylinderbereich und der wenigstens einen Wand verbunden ist, wobei die archimedische Schraube und die wenigstens eine Wand so angeordnet sind, so dass bei einer Drehung der Zellkultur-Flasche ein Fluid entlang der archimedischen Schraube von der Unterseite des Zylinderbereiches in den Deckelbereich gelangt.

Für das Rotationsmodul des erfindungsgemäßen Bioreaktors kann zweckmäßiger Weise auf ein Rollenmodul zurückgegriffen werden, umfassend mindestens zwei Rollen, ausgelegt und eingerichtet für Aufnahme und die Rotation des Reaktorgefäßes, insbesondere der Flasche, um dessen bzw. deren Längsachse, wobei das Rotationsmodul, insbesondere die mindestens zwei Rollen, gegenüber der Horizontalen in einem Neigungswinkel geneigt oder neigbar ist bzw. sind.

In einer besonders zuverlässig arbeitenden Ausführungsform umfasst das Rollenmodul vier Rollen, wobei je zwei Rollen paarweise zur Auflage des Reaktorgefäßes, insbesondere der Flasche, ausgelegt und eingerichtet sind und dass je zwei Rollen paarweise oberhalb des Reaktorgefäßes, insbesondere der Flasche, angeordnet sind.

Auch kann vorgesehen sein, dass das Rotationsmodul eine in Eingriff mit dem Reaktorgefäß bringbare Welle umfasst.

Ferner haben sich solche Ausführungsformen des erfindungsgemäßen Bioreaktors als besonders zweckmäßig erwiesen, bei denen das Agitationsmodul, insbesondere Vibrationsmodul, in dem Rotationsmodul integriert vorliegt, insbesondere in der mindestens einen Rolle, bevorzugt in mindestens einem Rollenpaar des Rollenmoduls.

Die der Erfindung zugrunde liegende Aufgabe wird ferner gelöst durch ein Prozessleitsystem umfassend mindestens einen erfindungsgemäßen Bioreaktor, insbesondere in Form einer Einwegkassette. Hierbei kann bevorzugt vorgesehen sein, dass das erfindungsgemäße Prozessleitsystem mit mindestens einer Inkubatoreinheit und/oder, insbesondere und, mindestens einem Pumpsystem ausgestattet ist.

Das erfindungsgemäße Prozessleitsystem umfasst in einer bevorzugten Ausführungsform ferner einen ersten Anschluss an die mindestens eine Leitung für den Transfer von Gasen oder Gasgemischen zu dem Reaktorgefäß mit mindestens der reversibel oder irreversibel verschließbaren Verschlusseinheit; einen zweiten Anschluss an die mindestens eine Leitung für den Transfer von Gasen oder Gasgemischen aus dem Reaktorgefäß mit mindestens der reversibel oder irreversibel verschließbaren Verschlusseinheit; einen dritten Anschluss an die mindestens eine Leitung für den Transfer flüssiger Systeme zu dem Reaktorgefäß mit mindestens einer reversibel oder irreversibel verschließbaren Verschlusseinheit und/oder einen vierten Anschluss an die mindestens eine Leitung für den Transfer flüssiger Systeme aus dem Reaktorgefäß mit mindestens einer reversibel oder irreversibel verschließbaren Verschlusseinheit. Insbesondere umfasst das erfindungsgemäße Prozessleitsystem einen dritten Anschluss an die mindestens eine Leitung für den Transfer flüssiger Systeme zu dem Reaktorgefäß mit mindestens einer reversibel oder irreversibel verschließbaren Verschlusseinheit und einen vierten Anschluss an die mindestens eine Leitung für den Transfer flüssiger Systeme aus dem Reaktorgefäß mit mindestens einer reversibel oder irreversibel verschließbaren Verschlusseinheit.

In einer besonders zweckmäßigen Ausführungsform umfasst das erfindungsgemäße Prozessleitsystem ferner mindestens einen Sensor, eingerichtet und ausgelegt zur Feststellung des Verbindungs- und/oder Arbeitsstatus des ersten, zweiten, dritten oder vierten Anschlusses mit dem jeweils korrespondierenden Verschlusselement.

Mit dem erfindungsgemäßen Bioreaktor bzw. dem erfindungsgemäßen Prozessleitsystem wird ohne weiteres eine GMP-konforme Analytik ermöglicht, beispielsweise hinsichtlich der Bestimmung des Gehalts an Glukose, Fruktose, Laktat, Sauerstoff und Kohlendioxid. Auch gelingt eine softwaregesteuerte Kontrolle sämtlicher Prozessparameter wie pH-Wert, Temperatur und Luftfeuchtigkeit. Von besonderem Vorteil ist zudem, dass der erfindungsgemäße Bioreaktor in Form einer Einwegkassette ohne weitere Reinigung und ohne weiteren Installationsaufwand in ein Prozessleitsystem enthaltend einen Inkubator bzw. ein Pumpsystem integriert werden kann. Ferner kann anhand der Auswertung der analytisch während des Betreibens des Bioreaktors ermittelten Parameter der weitere Verlauf der im Reaktorgefäß ablaufenden Prozesse feineingestellt bzw. gesteuert werden, beispielsweise durch gezielte Zugabe von Reaktionskomponenten wie Glukose.

Als besonders überraschend hat sich auch herausgestellt, dass mit den erfindungsgemäßen Bioreaktoren homogene Zellsuspensionen zuverlässig und wiederholbar erhältlich sind und dass bei der Ernte des generierten Zellmaterials Zellverklumpungen vermieden werden können.

Von besonderem Vorteil ist auch, dass Leckagen ausgeschlossen werden können und dass sämtliche beim erfindungsgemäßen Bioreaktor zum Einsatz kommenden Materialien chemisch inert ausgeführt werden können und zudem ohne weiteres sterilisierbar sind.

Mit dem erfindungsgemäßen Bioreaktor kann sichergestellt werden, dass bei der Herstellung der vermehrten Zellen (z.B. HCT/Ps wie mesenchymale Stammzellen, induzierte pluripotente Stammzellen, primäre Zellen und Zelllinien) das Risiko der Einführung, Übertragung oder Ausbreitung übertragbarer Krankheiten extrem gering gehalten wird bzw. komplett ausgeschlossen werden kann.

Weitere Merkmale und Vorteile der Erfindung ergeben sich aus der nachgehenden Beschreibung, in der bevorzugte Ausführungsformen der Erfindung beispielhaft anhand schematischer Zeichnungen erläutert sind. Dabei zeigen:
- Figur 1: Komponenten des erfindungsgemäßen Bioreaktors in perspektivischer Darstellung;
- Figur 2: eine Querschnittsansicht eines erfindungsgemäßen Bioreaktors;
- Figur 3: Komponenten eines erfindungsgemäßen Prozessleitsystems in schematischer Darstellung; und
- Figur 4: eine Querschnittsansicht von Komponenten eines erfindungsgemäßen Bioreaktors.

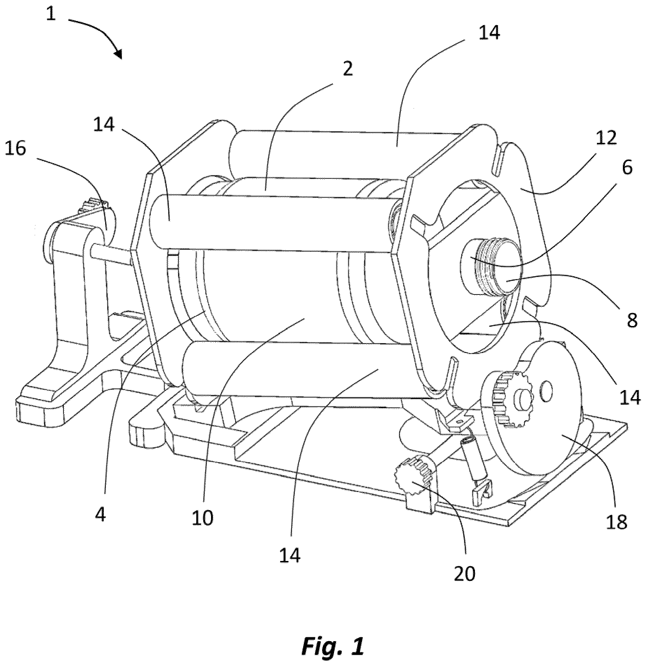

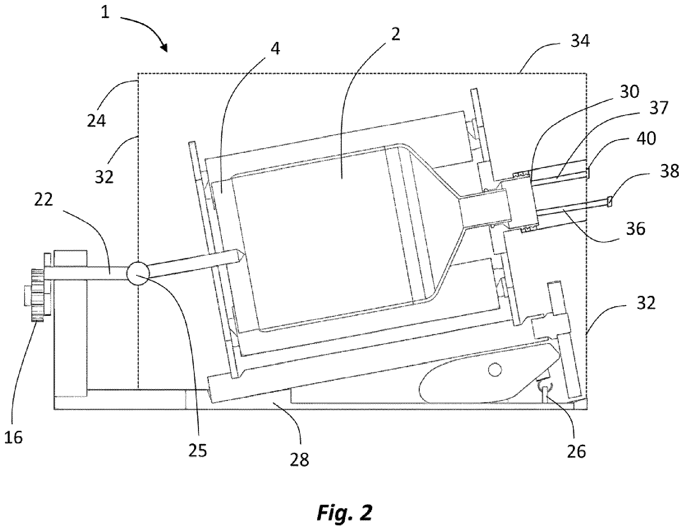

Figur 1 zeigt Komponenten des erfindungsgemäßen Bioreaktors (1) in perspektivischer Darstellung. Das Reaktorgefäß (2) verfügt über einen Reaktorboden (4) und ein gegenüberliegendes Reaktorende (6). Das Reaktorgefäß (2) bzw. die sich von dem Reaktorboden (4) bis zum gegenüberliegenden Reaktorende (6) erschreckende Längsachse ist bei gattungsgemäßer Verwendung, wie in der dargestellten Ausführungsform gezeigt, gegenüber der Horizontalen geneigt, sodass das dem Reaktorboden (4) gegenüberliegende Reaktorende (6) weiter beabstandet von der Horizontalen ist. An dem Reaktorboden (4) gegenüberliegenden Reaktorende (6) liegt ein Reaktoreinlass (8) vor. Das Reaktorgefäß (2) hat in der dargestellten Ausführungsform die Form einer Zellkultur-Flasche. Die Seitenwand (10) dieses Reaktorgefäßes (2) wird von vier in einem Rahmen (12) vorliegenden drehbaren Rollen (14) gehalten. Das flaschenförmige Reaktorgefäß (2) liegt hierbei auf dem unteren Rollenpaar (14) auf. Die Rollen (14) sind, ebenso wie der Rahmen (12), Bestandteil des Rotationsmoduls (15) des erfindungsgemäßen Bioreaktors. Die Rotation des Reaktorgefäßes (2) wird dabei durch eine in der dargestellten Ausführungsform mit dem Reaktorboden (4) über ein in der Gehäusewandung vorliegendes Kupplungsgelenk (nicht abgebildet; siehe Figur 2) verbundene und in Teilen dargestellte Antriebseinheit (16) herbeigeführt. Darüber hinaus verfügt der erfindungsgemäße Bioreaktor über eine Agitationseinheit bzw. -modul (18), die in der dargestellten Ausführungsform eine Exenterscheibe beinhaltet. Diese Agitationseinheit (18) ist mit einem Antrieb (nicht abgebildet) verbunden bzw. verbindbar und wird dazu benutzt, das Reaktorgefäß (2) in periodischen Abständen mechanisch zu erschüttern, beispielsweise um die Ernte von in dem Reaktorgefäß (2) kultivierten Zellen zu initiieren bzw. zu forcieren. Der Grad der Neigung des Reaktorgefäßes (2) kann durch eine Höhenverstelleinheit (20) eingestellt werden.

Figur 2 zeigt eine Querschnittsansicht durch eine Vorrichtung, die im Wesentlichen der Vorrichtung gemäß Figur 1 entspricht (In Figur 1 ist z.B. das Gehäuse 24 nicht mit eingezeichnet). Die Welle (22) von der Antriebseinheit (16) für das Rotationsmodul (15) zu der Anbindung an den Boden (4) des Reaktorgefäßes (2) mündet in der dargestellten Ausführungsform in eine in der Gehäusewandung des Gehäuses (24) des erfindungsgemäßen Bioreaktors vorliegende Gelenkkupplung (25). Über diese Gelenkkupplung kann die Antriebseinheit (16) von dem Rotationsmodul (15) entkoppelt werden. Auch ist der Figur 2 eine Federaufhängung (26) in Verbindung mit der Bodenplatte (28) des Gehäuses (24) des Reaktorgefäßes (2) zu entnehmen. Auf diese Weise wird in Wechselwirkung mit der Bewegung der Exzenterscheibe eine nochmals stärkere Erschütterung des Reaktorgefäßes (2) herbeigeführt. In der Figur 2 sind die Seitenwände (32) und die obere Abdeckung (34) der Gehäusewandung des Gehäuses (24) durch gestrichelte Linien angedeutet. Am Reaktoreinlass (30) des Reaktorgefäßes (2) liegt eine Leitung (36) für den Transfer flüssiger Systeme vor, die sich bis über die Gehäusewandung (32) hinaus erstreckt und dort in eine Verschlusseinheit (38) in Form eines Sterilkonnektors mündet bzw. einen Sterilkonnektor umfasst. Weiterhin erstreckt sich in der dargestellten Ausführungsform von dem Reaktoreinlass (30) bis zur Gehäusewandung (32) eine Leitung (37) für den Transfer von Gasen bzw. Gasgemischen, welche in eine Verschlusseinheit (40) in Form eines Sterilfilters mündet bzw. übergeht. Auf diese Weise kann bei dem erfindungsgemäßen Bioreaktor ein nicht öffenbares Gehäuse (24) zum Einsatz kommen, das darüber hinaus gas- und flüssigkeitsdicht ist.

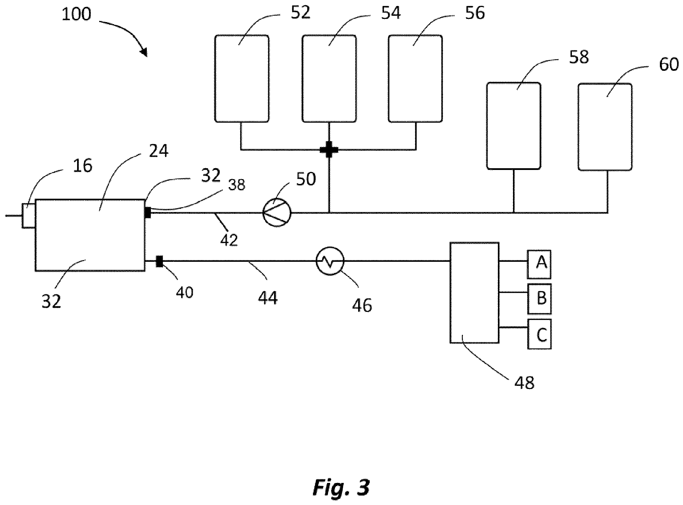

Figur 3 zeigt Komponenten einer Ausführungsform des erfindungsgemäßen Prozessleitsystems (100) in schematischer Darstellung. Nicht expliziert dargestellt werden dabei die Komponenten des in dem Gehäuse (24) vorliegenden erfindungsgemäßen Bioreaktors (1). An das Gehäuse (24) bzw. an die rückseitige Gehäusewandung (32) setzt die Antriebseinheit (16) des Rotationsmoduls an. Frontseitig befindet sich integriert in die Gehäusewandung (32) ein Sterilkonnektor (40), an den sich eine Leitung (42) für den Transfer flüssiger Systeme anschließt. Ebenfalls ist ein Sterilfilter (40) in der frontseitigen Gehäusewandung (32) bzw. - in der dargestellten Ausführungsform - an dem Ende einer außenliegenden, von der Gehäusewandung ausgehenden oder an dem Ende einer die Gehäusewandung durchtretenden Leitung vorgesehen. An diesen Sterilfilter schließt sich eine Leitung (44) für den Transfer von Gasen bzw. Gasgemischen an. Die über diese Transferleitung (44) in das Gehäuse bzw. in das Reaktorgefäß einleitbaren Gase und Gasgemische können über eine mit dieser Transferleitung verbundene Temperiereinheit (46) vor Einleitung in das Reaktorgefäß aufgeheizt werden. Über ein in der dargestellten Ausführungsform ebenfalls anwesendes Mischaggregat (48) können zwei oder mehr Gase (A, B, C) vor Einleitung in das Gehäuse (24) bzw. das Reaktorgefäß (2) homogen vermischt werden.

In der sich an den Sterilkonnektor (38) außerhalb des Gehäuses anschließenden Transferleitung (42) für flüssige Systeme ist eine Pumpeinheit (50) vorgesehen, die sowohl dafür ausgelegt ist, wässrige Systeme wie Reaktormedium (52), Waschlösung (54) und Erntereagenz (56) aus separat vorliegenden Gebinden in das Reaktorgefäß (2) zu überführen, als auch um die in dem Reaktorgefäß (2) kultivierten Zellen nach Beendigung der Kultivierungsperiode in ein entsprechendes Erntegebinde (58) zu überführen. Ferner erlaubt es das erfindungsgemäße Prozessleitsystem (100), das Reaktorgefäß (2) nach erfolgter Ernte der kultivierten Zellen mit einer Waschlösung aus einem separaten Gebinde (54) zu reinigen und die hierbei anfallenden Flüssigkeiten gezielt in einem separaten Gebinde (60) aufzunehmen, um sie sodann fachgerecht zu entsorgen. Nach beendeter Isolierung der kultivierten Zellen sowie Reinigung des Reaktorgefäßes (2) kann der Bioreaktor vom Prozessleitsystem (100) abgekoppelt und einer separaten Entsorgung bzw. einem separaten Recyclingprozess zugeführt werden.

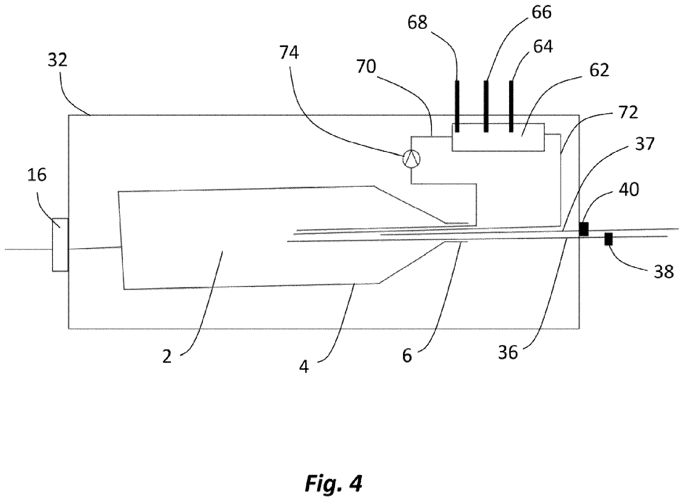

Figur 4 entnimmt man eine Querschnittsansicht von Komponenten eines erfindungsgemä-ßen Bioreaktors. In dem Gehäuse (32) liegt das Reaktorgefäß (2) in Form einer Zellkultur-Flasche vor. Das Rotations- und das Agitationsmodul bzw. deren Komponenten sind in Figur 4 der besseren Übersicht wegen nicht gezeigt. Durch den Reaktoreinlass (30) des Reaktorgefäßes (2) führt eine Leitung (36) für den Transfer flüssiger Systeme. Diese erstreckt sich bis über die Gehäusewandung (32) hinaus und geht dort in eine Verschlusseinheit (38) enthaltend einen Sterilkonnektor über. Die Leitung (36) erstreckt sich bis in den mittleren Teil des Reaktorgefäßes (2) hinein. Auf diese Weise kann sichergestellt werden, dass diese Leitung während des Einsatzes des Biorektors stets in das Kulturmedium eintaucht. Die Leitung (37) für den Transfer von Gasen bzw. Gasgemischen mündet in eine in der Gehäusewandung (32) vorliegende Verschlusseinheit (40) enthaltend einen Sterilfilter (40). Diese Leitung erstreckt sich nur geringfügig über den Reaktoreinlass (30) hinaus. Hierdurch wird gewährleistet, dass diese Leitung während des Einsatzes des Biorektors nicht in das Kulturmedium eintaucht. Bestandteil der in Figur 4 dargestellten Ausführungsform eines erfindungsgemäßen Bioreaktors ist ebenfalls eine Messzelle (62), die innerhalb des Gehäuses (24) vorliegt. Dieser Messzelle wird über die Flüssigleitung 70 mit Hilfe einer Pumpeinheit (74) Kulturmedium aus dem Reaktorgefäß (2) zugeführt. Nach erfolgter Vermessung des Probenmaterials wird dieses durch die Leitung (72) wieder rücküberführt in das Reaktorgefäß (2). Vorteilhafterweise handelt es sich bei der Messzelle um eine sogenannte Durchflusszelle. Die Pumpeinheit (74) kann z.B. als Schlauchpumpe ausgeführt sein, wobei der Pumpenantrieb außerhalb des Gehäuses vorliegt und durch eine Antriebswelle mit den Pumpenelementen der Schlauchpumpe in Wirkverbindung steht. Zweckmäßigerweise liegt die Messzelle (62) an bzw. im Bereich der Innenwandung des Gehäuses (24) vor. In der dargestellten Ausführungsform verfügt die Messzelle (62) über einen Sauerstoffsensor (68), einen pH-Sensor (66) und einen Glukose-Sensor (64). Diese Sensoren sind ausgelegt und eingerichtet, um die detektierten Parameter z.B. an das Prozessleitsystems (100) weiterzuleiten. Auf diese Weise können Anpassungen an die Reaktionsparameter während des Kultivierungsprozesses vorgenommen werden, um zu einer optimierten Ausbeute zu gelangen.

Die in der vorstehenden Beschreibung, in den Ansprüchen und in den Zeichnungen offenbarten Merkmale der Erfindung können sowohl einzeln aus auch in jeder beliebigen Kombination für die Verwirklichung der Erfindung in ihren verschiedenen Ausführungsformen wesentlich sein.

## Patentansprüche

1. Bioreaktor (1), insbesondere Single-use Bioreaktor, zum Kultivieren von, insbesondere adhärenten, Zellen, umfassend
- ein Gehäuse (24) mit einer Gehäusewandung (32) und einem Gehäuseboden (28) sowie
- ein Reaktorgefäß (2) mit einem Reaktorboden (4) und einem gegenüberliegenden Reaktorende (6) sowie einer sich von dem Reaktorboden (4) zum gegenüberliegenden Reaktorende (6) erstreckenden Längsachse, wobei vorzugsweise die Längsachse gegenüber der Horizontalen einen Neigungswinkel aufweist,
- mindestens eine Leitung (37) für den Transfer von Gasen oder Gasgemischen zu dem und/oder aus dem Reaktorgefäß (2) mit mindestens einer reversibel oder irreversibel verschließbaren Verschlusseinheit (40),
- mindestens eine Leitung (36) für den Transfer flüssiger Systeme zu dem und/oder aus dem Reaktorgefäß (2) mit mindestens einer reversibel oder irreversibel verschließbaren Verschlusseinheit (38) und
- mindestens ein Rotationsmodul (15), ausgelegt und eingerichtet für die Bewegung des Reaktorgefäßes (2) um die Längsachse.

2. Bioreaktor (1) nach Anspruch 1, ferner umfassend
mindestens ein, insbesondere in dem Gehäuse (24) vorliegendes, Agitationsmodul (18), ausgelegt und eingerichtet für die Erschütterung des Reaktorgefäßes (2), wobei das Agitationsmodul (18) bevorzugt ein Vibrationsmodul darstellt oder umfasst und besonders bevorzugt eine Exzenterscheibe darstellt oder umfasst.

3. Bioreaktor (1) nach Anspruch 1 oder 2, ferner umfassend
mindestens eine in dem Gehäuse (24) oder vorzugsweise außerhalb des Gehäuses (24) vorliegende erste Antriebseinheit (16), ausgelegt und eingerichtet zum Antrieb des Rotationsmoduls (15) und/oder, insbesondere und, mindestens eine in dem Gehäuse (24) oder vorzugsweise außerhalb des Gehäuses vorliegende zweite Antriebseinheit, ausgelegt und eingerichtet zum Antrieb des Agitationsmoduls (18).

4. Bioreaktor (1) nach einem der vorangehenden Ansprüche, **dadurch gekennzeichnet, dass**
die mindestens eine Leitung (37) für den Transfer von Gasen oder Gasgemischen zu dem und/oder aus dem Reaktorgefäß (2) mit der mindestens einen Verschlusseinheit (40) in einen in der oder benachbart zur Gehäusewand vorliegenden ersten Sterilkonnektor mündet und/oder, insbesondere oder, dass die mindestens eine Leitung (37) für den Transfer von Gasen oder Gasgemischen zu dem und/oder aus dem Reaktorgefäß (2) mit der mindestens einen Verschlusseinheit (40), insbesondere benachbart zur Gehäusewand, mit einem Sterilfilter ausgestattet ist und/oder, insbesondere und, dass die mindestens eine Leitung (36) für den Transfer flüssiger Systeme zu dem und/oder aus dem Reaktorgefäß (2) mit der mindestens einen Verschlusseinheit (38) in einen in der oder benachbart zur Gehäusewand vorliegenden zweiten Sterilkonnektor mündet.

5. Bioreaktor (1) nach einem der vorangehenden Ansprüche, ferner umfassend mindestens eine interne oder externe, insbesondere strom-, flüssigkeits- oder gasbasierte, Temperiereinheit (46).

6. Bioreaktor (1) nach einem der vorangehenden Ansprüche, ferner umfassend, insbesondere in dem Gehäuse (24) oder in dem Reaktorgefäß (2) vorliegend,
mindestens einen Sauerstoffsensor und/oder
mindestens einen Kohlendioxidsensor und/oder
mindestens einen Laktatsensor und/oder
mindestens einen Glukosesensor und/oder
mindestens einen Fruktosesensor und/oder
mindestens einen Temperatursensor und/oder
mindestens einen pH-Sensor und/oder
mindestens einen Impedanzsensor und/oder
mindestens einen Permittivitätssensor und/oder
mindestens einen Kapazitätssensor und/oder
mindestens einen Kontaktsensor eingerichtet und ausgelegt zur Feststellung des Öffnungsstatus der mindestens einen Verschlusseinheit (38, 40).

7. Bioreaktor (1) nach einem der vorangehenden Ansprüche, ferner umfassend mindestens eine erste Pumpeinheit oder ein erstes Pumpenelement in Wirkverbindung mit der mindestens einen Leitung (37) für den Transfer von Gasen oder Gasgemischen zu dem und/oder aus dem Reaktorgefäß (2) und/oder mindestens eine zweite Pumpeinheit oder ein zweite Pumpenelement (50) in Wirkverbindung mit der mindestens einen Leitung (36) für den Transfer flüssiger Systeme zu dem und/oder aus dem Reaktorgefäß (2),
wobei die mindestens eine zweite Pumpeinheit vorzugsweise ausgelegt und eingerichtet ist für die Entnahme und/oder Zugabe von Prozessmedien und/oder Reagenzien und/oder kultivierten Zellen.

8. Bioreaktor (1) nach einem der vorangehenden Ansprüche, ferner umfassend eine innerhalb oder außerhalb des Gehäuses (24) vorliegende Höhenverstelleinheit (20) zur Einstellung des Neigungswinkels der Längsachse des Reaktorgefäßes (2), wobei die mindestens eine Pumpeinheit vorzugsweise ausgelegt und eingerichtet ist für die Entnahme und/oder Zugabe von Prozessmedien und/oder Reagenzien und/oder kultivierten Zellen.

9. Bioreaktor (1) nach einem der vorangehenden Ansprüche, **dadurch gekennzeichnet, dass**
das Rotationsmodul (15) ein Rollenmodul umfasst oder darstellt, umfassend mindestens zwei Rollen (14), ausgelegt und eingerichtet für Aufnahme und die Rotation des Reaktorgefäßes (2), insbesondere der Flasche, um dessen bzw. deren Längsachse, wobei das Rotationsmodul (15), insbesondere die mindestens zwei Rollen (14), gegenüber der Horizontalen in einem Neigungswinkel geneigt oder neigbar sind,
oder dass das Rotationsmodul (15) eine in Eingriff mit dem Reaktorgefäß (2) bringbare Welle umfasst,
wobei das Rollenmodul vorzugsweise vier Rollen (14) umfasst, wobei je zwei Rollen (14) paarweise zur Auflage des Reaktorgefäßes (2), insbesondere der Flasche, ausgelegt und eingerichtet sind und dass je zwei Rollen (14) paarweise oberhalb des Reaktorgefäßes (2), insbesondere der Flasche, angeordnet sind.

10. Bioreaktor (1) nach einem der vorangehenden Ansprüche, **dadurch gekennzeichnet, dass**
das Agitationsmodul (18), insbesondere reversibel, mit dem Gehäuse (24) verbunden oder verbindbar ist.

11. Bioreaktor (1) nach einem der vorangehenden Ansprüche, **dadurch gekennzeichnet, dass**
die mindestens eine Leitung (37) für den Transfer von Gasen oder Gasgemischen zu dem und/oder aus dem Reaktorgefäß (2) eine Luft-, Sauerstoff-, Stickstoff- und/oder eine Kohlendioxidzuleitung zu dem Reaktorgefäß (2) darstellt.

12. Bioreaktor (1) nach einem der vorangehenden Ansprüche, **dadurch gekennzeichnet, dass**
die mindestens eine Leitung (36) für den Transfer flüssiger Systeme aus dem Reaktorgefäß (2), insbesondere aus der Flasche, eine Abführleitung für kultivierte Zellen darstellt oder umfasst.

13. Bioreaktor (1) nach einem der vorangehenden Ansprüche, **dadurch gekennzeichnet, dass**
in den Leitungen (37) für den Transfer von Gasen oder Gasgemischen mindestens ein Sterilfilter vorgesehen oder integriert ist und/oder dass in den Leitungen (36) für den Transfer flüssiger Systeme mindestens ein Sterilkonnektor vorgesehen oder integriert ist.

14. Bioreaktor (1) nach einem der vorangehenden Ansprüche, **dadurch gekennzeichnet, dass**
das Gehäuse, insbesondere die Gehäusewandung (32), mit mindestens einem Sterilfilter ausgestattet ist, ausgelegt und eingerichtet für den Abbau von Überdruck in dem Gehäuse.

15. Bioreaktor (1) nach einem der vorangehenden Ansprüche, ferner umfassend mindestens eine in Fluidverbindung mit dem Reaktorgefäß stehende, vorzugsweise in dem Gehäuse (24) vorliegende, Messzelle (62), insbesondere Durchflussmesszelle, enthaltend mindestens einen Sensor, bevorzugt einen Sauerstoff-, Kohlendioxid-, Laktat-, Glukose-, Fruktose-, pH-Wert-, Impedanz-, Permittivitäts- oder Kapazitätssensor, besonders bevorzugt einen Sauerstoff-, Kohlendioxid-, Laktat-, Glukose-, Fruktose- oder pH-Wert-Sensor.

16. Prozessleitsystem umfassend mindestens einen Bioreaktor (1) nach einem der vorangehenden Ansprüche.

17. Prozessleitsystem (100) nach Anspruch 16, ferner umfassend
ein Aufnahmemodul für den Bioreaktor (1).

18. Prozessleitsystem (100) nach Anspruch 16 oder 17, ferner umfassend
einen ersten Anschluss an die mindestens eine Leitung (37) für den Transfer von Gasen oder Gasgemischen zu dem Reaktorgefäß (2) mit mindestens der reversibel oder irreversibel verschließbaren Verschlusseinheit (40),
einen zweiten Anschluss an die mindestens eine Leitung (37) für den Transfer von Gasen oder Gasgemischen aus dem Reaktorgefäß (2) mit mindestens der reversibel oder irreversibel verschließbaren Verschlusseinheit (40),
einen dritten Anschluss an die mindestens eine Leitung (36) für den Transfer flüssiger Systeme zu dem Reaktorgefäß (2) mit mindestens einer reversibel oder irreversibel verschließbaren Verschlusseinheit (38) und/oder
einen vierten Anschluss an die mindestens eine Leitung (36) für den Transfer flüssiger Systeme aus dem Reaktorgefäß (2) mit mindestens einer reversibel oder irreversibel verschließbaren Verschlusseinheit (38).

19. Prozessleitsystem (100) nach Anspruch 18, ferner umfassend
mindestens einen Sensor, eingerichtet und ausgelegt zur Feststellung des Verbindungs- und/oder Arbeitsstatus des ersten, zweiten, dritten oder vierten Anschlusses mit dem jeweils korrespondierenden Verschlusselement.
